# EUROPEAN PATENT SPECIFICATION

(11) **EP 2 943 410 B1**
(45) Date of publication and mention of the grant of the patent: **28.03.2018**
(21) Application number: 13863812.7
(22) Date of filing: 19.12.2013
(51) Int. Cl.: B65D 5/42, B65B 31/04, B65B 57/00, G01N 21/39, G01N 21/90, G01N 33/14

(54) **A METHOD FOR MANUFACTURING OF A CONTAINER FOR FOOD PRODUCTS**
VERFAHREN ZUR HERSTELLUNG EINES LEBENSMITTELBEHÄLTERS
PROCÉDÉ DE FABRICATION D'UN CONTENANT POUR PRODUITS ALIMENTAIRES

(30) Priority: 21.12.2012 SE 1251491
(43) Date of publication of application: 18.11.2015
(73) Proprietor: Å&R Carton Lund AB, 221 00 Lund (SE)
(72) Inventor: HOLKA, Simon, S-245 91 Staffanstorp (SE); AVELING, Lennart, S-236 36 Höllviken (SE)
(74) Representative: Valea AB
(86) International application number: PCT/SE2013/051580
(87) International publication number: WO 2014/098757

(56) References cited:
- EP-A1- 2 447 175
- EP-A2- 0 074 343
- EP-A2- 1 361 161
- WO-A1-2005/113347
- WO-A1-2010/145892
- DE-A1- 3 920 484
- DE-U1- 20 311 488
- DE-U1- 20 311 488
- DE-U1-202007 007 396
- DE-U1-202007 007 396
- JP-A- 2002 080 028
- JP-A- 2002 080 028
- US-A1- 2009 297 741
- US-B1- 6 213 387

## Description

### TECHNICAL FIELD

This invention relates to a method for manufacturing of a container for food products or other sensitive products from blanks of a cardboard based material. The container disclosed herein is made of a material that intends to provide the container with gas-tight properties and that comprises a layer that exhibits no or only a low translucence to light. At least one opening is provided in the layer with no or low light translucence such as to allow light for non-destructive detection of gas inside the container to enter and exit through said at least one opening. Disclosed herein is also a method for detecting gas inside a sealed container of the above type. Disclosed herein is also a production system comprising an apparatus for filling containers of the above type with a product and an apparatus for sealing the filled containers, wherein the system further comprises an apparatus for carrying out the method for detecting gas inside the sealed container.

### BACKGROUND OF THE INVENTION

Many food products are packaged in sealed gas-tight containers with a controlled internal atmosphere as to protect the food stuff and prolong shelf-life etc. Typically, a headspace of the food container, i.e. the space inside the container above the food and below the seal, is evacuated or contains close to 100% nitrogen gas to avoid the presence of oxygen gas.
For quality and safety reasons it is sometimes necessary to check the status of the internal atmosphere that is in contact with the food product. Traditionally, the methods for this purpose involve puncturing the container which leads to waste of both products and packages.
In an alternative method described in WO2010/145892 a laser source is used to direct light through the container wall and into (the headspace of) the package. Scattered light enters the package and interacts with gas inside the package. By measuring, in a position outside of the package, an absorption signal of the light that exits the package it is possible to measure e.g. the concentration of oxygen gas inside the package without puncturing it. Only the light, incoming and outgoing, needs to pass through the walls or lid of the package. The package may appear non-transparent but it is sufficient that the package is translucent to a certain wavelength range of the laser light.
The method of WO2010/145892 is very useful in many applications but is not applicable to containers made of a material that is not translucent to light, such as a multilayer laminate where an aluminium foil forms one of the layers. Container materials of this type include e.g. cardboard laminates used for food in dry powder form.

JP 2002 080028 A discloses a container for a liquid extracorporeal diagnosis reagent and a method for manufacturing of a container according to the preamble of claim 1. The container is made of a laminated paper base material comprising polymer films and an aluminium layer and is provided with transparent film windows (peep windows).
DE 203 11 488 U1 discloses an aluminium lined fluid container which is provided with a fluid level viewing window in a side wall. A method for manufacturing the container is also disclosed.
EP 1 361 161 A2 discloses a multi-ply, spiral-wound container having a window formed in a body ply and being covered by a low opacity liner ply, thereby allowing a product in the container to be viewed through the window.
There is thus a need for improvements with regard to methods for forming a container allowing checking of the status of the internal atmosphere in containers made of a material that is not translucent to light.

### SUMMARY OF THE INVENTION

The invention concerns a method for manufacturing a container for food products or other sensitive products, in accordance with claim 1. The container as disclosed herein is made of a material that intends to provide the container with gas-tight properties and that comprises a layer that exhibits no or only a low translucence to light.
At least one opening is provided in the layer with no or low light translucence, such as to allow light for nondestructive detection of gas inside the container to enter and exit through said at least one opening.

Thereby, laser light can be allowed to enter the (headspace of the) container, interact with the gas present (if any) and exit via the same or another opening and be detected and analyzed outside of the container such as to check the content and concentration of the gas present. The technique described in WO2010/145892 is applicable but also other techniques can be used as the light does not have to be scattered when passing into the container.
Typically, the container has a bottom, walls and a sealing membrane or sealed lid on top.
The term gas-tight properties is used in the common-sense meaning, i.e. it means as gas-tight as reasonably achievable. No material is 100% gas-tight overtime.

In an example of the container as disclosed herein, the opening is covered with a covering material that has a high translucence to light. The covering material may thus be transparent to light.
Preferably, the covering material forms gas barrier and/or the at least one opening is sufficiently small so as to avoid that the overall gas-tightness of the container is significantly reduced. Thus, a container that has a small opening covered with e.g. a layer of plastic material can still be "gas-tight".
Using a covering material makes it possible to use (a) larger opening(s). In general terms, an opening with a side/diameter of around 0.1 mm can be used without covering material in a container having a volume of 1 liter. Such an opening may be too small to allow enough light to exit for the analysis of the internal gas atmosphere. Openings/windows in the size 2x2 mm or 4x4 mm may be sufficiently large for some gas detection techniques and may be covered by regular container multilayer plastic material depending on the type of material and the size of the container.
An opening size of at least 4x20 mm is required for some light reflection techniques. Such large openings need to be covered with a special material, i.e. a material with special gas permeability and/or special thickness. Using a special plastic material over the entire container structure would be too costly. It is possible to locally weld the special plastic material over the openings but such an additional production step is also costly. A further method is described below.

Preferably, at least a part of the at least one opening is positioned in an upper position of the container in a region intended to contain an internal gas atmosphere above a product placed in the container. Thereby, light can be directed into the headspace when a filled container is placed in a normal position with its bottom facing downwards. The at least one opening is arranged in a wall section of the container. As a complement, an opening can be placed in the sealing lid.

In a typical example of the container as disclosed herein, the container is made of multilayer structure comprising a layer of aluminium, wherein the at least one opening is arranged in the aluminium layer. The aluminium layer preferably has a thickness in the range 4-40 pm. This makes the aluminium layer gas-tight and non-translucent to light.
Such a multilayer structure preferably also comprises a layer of a weldable plastic material that also forms the covering layer. The opening(s) can thus be made in the aluminium layer before laminating the aluminium and plastic layers to each other, during which process the covering layer will form without any special step. The covering layer can alternatively be formed by attaching a tape or similar to the laminate after having arranged the opening in the entire multilayer structure.
Preferably, the multilayer structure also comprises a layer of a cardboard material that works as a supporting layer.
A method for determining a gas content in a container according to what is described above is also disclosed herein. This method comprises the steps of directing light through the at least one opening into the container, and detecting light that exits from the container through the at least opening.
In variants of the gas content determining method the following may be applied:
- the light directed into the container is emitted from a laser source,
- the absorption of the light that exits the container is measured,
- the container comprises more than one opening and the light enters the container through a first opening and exits the container through a second opening.

The invention concerns a method for manufacturing of a container according to above from blanks of a cardboard based material, said method comprising the steps of:
- providing a substantially plane blank of a multilayer material comprising a supporting cardboard layer, a weldable layer and a gas-tight layer,
- arranging, in at least the cardboard layer, at least one cut-out,
- forming a rounded container body from the blank by bending the blank such as to connect said first edge with an opposite edge of the blank wherein a joint is formed along the connected edges,
- applying a gas-tight and translucent strip of material in such a way that the opening is covered by the material strip.

The at least one cut-out is arranged at the first edge of the blank, wherein an opening is formed at the at least one cut-out when forming the rounded container body.

Also disclosed herein is a production system comprising i) an apparatus for filling containers according to above with a product and ii) an apparatus for sealing the filled containers, wherein the system further comprises iii) an apparatus for carrying out the method for detecting gas inside the sealed container.

### BRIEF DESCRIPTION OF DRAWINGS

In the description of the invention given below reference is made to the following figures, in which:
- Figure 1: shows, in a schematic view, a container according to an embodiment of the present disclosure,
- Figure 2: shows, in a schematic view, the principle of a first embodiment of a gas detection method applied to a container similar to what is shown in figure 1,
- Figure 3: shows, in a schematic view, the principle of a second embodiment of a gas detection method applied to a container similar to what is shown in figure 1, and
- Figures 4a and 4b: show examples of a blank provided with a cutout/opening.

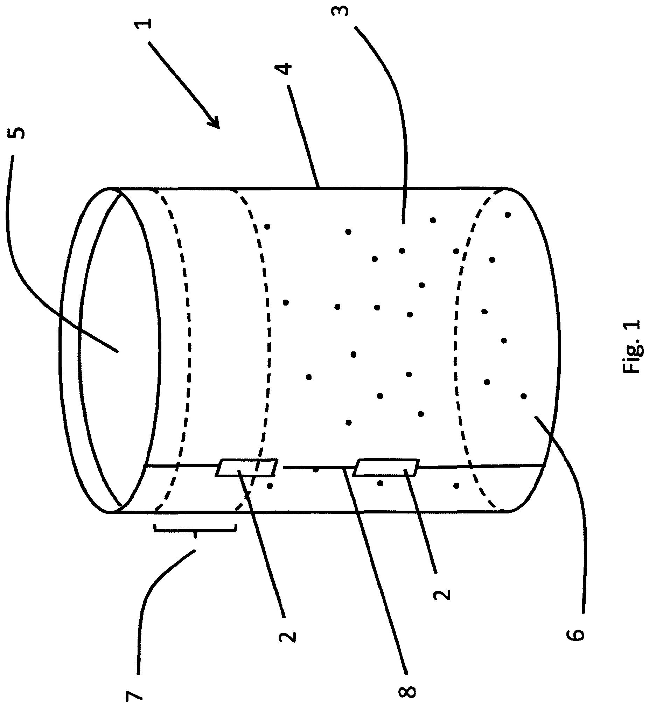

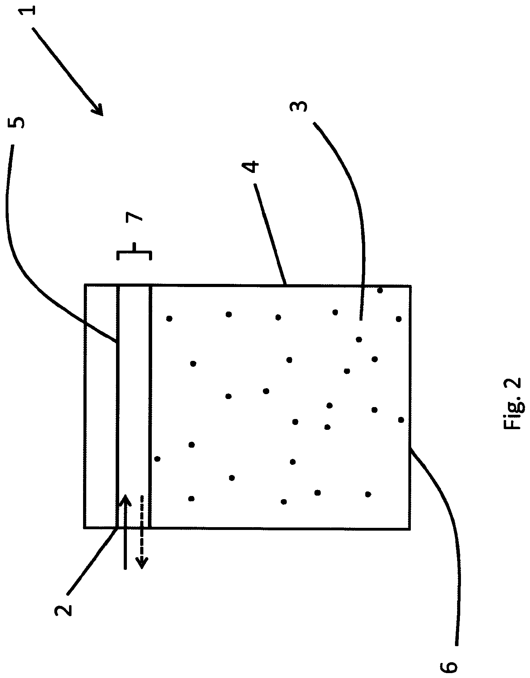

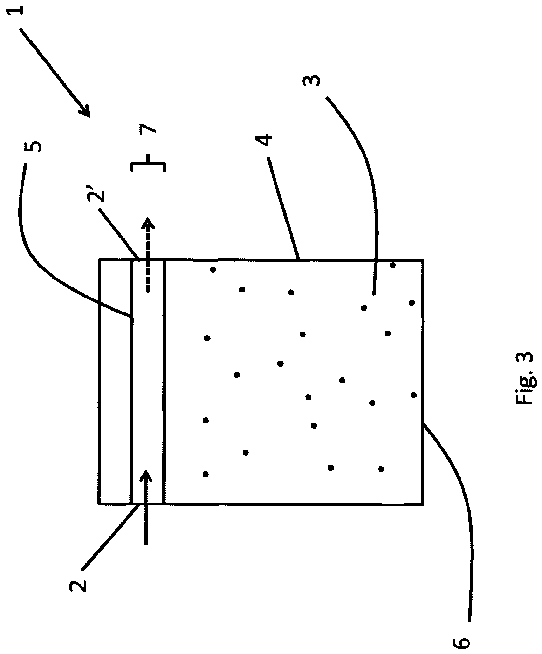

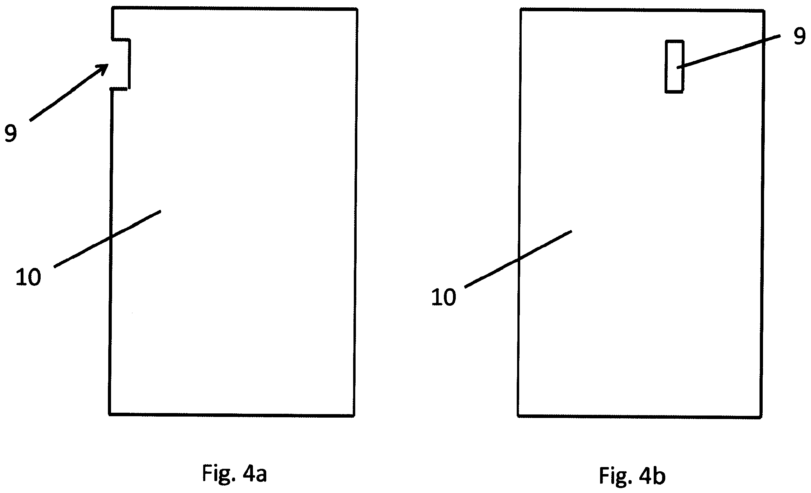

The container shown in Fig. 1 is only part of the present invention when being a product of the method according to claim 1 or any one of the dependent claims. The gas detection methods shown in Figs. 2 and 3 and described herein do not form part of the invention. The blanks shown in Figs. 4a and 4b and described herein do not form part of the invention.

### DESCRIPTION OF EXAMPLE EMBODIMENTS

Equipment for manufacturing of containers from blanks of a cardboard based material is described in e.g. EP0074343. Such equipment normally includes a container body forming unit where an initially cylindrical container body is formed from a substantially plane blank of a multilayer material comprising at least a supporting cardboard layer and a weldable, plastic layer. In a welding unit, an end closure, a bottom, is applied to the inside of the container body by inducing welding energy and melting the weldable layer. Such a welded joint is well known to be capable of being gas tight. After welding the bottom edge of the container body is normally processed in an edge shaping unit where the edge typically is folded, heated and curled to provide stability and a more appealing appearance. Also the body blank may be welded together as to form the cylindrical container body, but welding of the end closure is usually a more complex process step.

Often both the container body and the end closure form multilayer structures where a weldable plastic film is applied to the inside of the cardboard based lid and container. In particular for food products, the layer structure usually includes a layer of aluminium arranged between the cardboard and the plastic layer. Typically, a high frequency current energy is induced in the aluminium layer, which foil thereby gets heated and in turn melts the plastic film. Automated equipment of this type further comprises transporting means configured to transport a flow of containers from the container body forming unit to the welding unit and further downstream in the equipment.

Equipment for filling the containers and for sealing and closing the filled containers can be arranged in connection to the container manufacturing equipment.

Instead of using aluminium for the gas-tight layer it is possible to use a metalized film. Such a film can be adapted to exhibit a sufficient light translucence for detection purposes.

The equipment and material described above are suitable for the container as described herein. By providing one or both of the edges of the body blank with one or several cut-outs, one or several openings will form when the two edges are joined to each other, i.e. when the rounded container body is formed. By covering the joint or seam and the openings formed between the edges with a strip of material that exhibits gas-barrier properties and that is translucent and maybe also transparent to (laser) light, one or several useful windows (for later detection of gas inside the container) are formed along the joint. This strip of material can be a tape, i.e. provided with an adhesive, and/or be welded to the two edges as to form a gas tight joint.

The remaining manufacturing of the container can follow the conventional method.

A schematic view of a container 1 of the type described above is shown in figure 1. The container 1 has a rounded wall section 4 formed by bending a blank as to form a joint 8 between edges of the wall section 4. Cut-outs have been punched out at both edges such that oblong openings 2 are formed along the joint 8. A gas-tight and translucent strip of material (not shown) has been applied along the joint 8 and covers the openings 2. The container 1 has further a bottom closure 6 and has been filled with food product powder 3. A sealing membrane 5 seals an upper part of the container 1. A headspace 7 is formed between the powder 3 and the sealing membrane 5.

The material forming the wall section 4 and the bottom 6 is a multilayer material comprising a supporting cardboard layer, a weldable layer and an aluminium layer. The aluminium layer or foil has typically a thickness of 6.35 or 9 pm. Al-foils with larger thickness are sometimes used, e.g. 18 or 35-38 pm. Generally, the Al-layer can be said to a have thickness of 4-40 pm.

The method for manufacturing of the container 1 from blanks of a cardboard based material comprises the steps of:
- providing a substantially plane blank of a multilayer material comprising a supporting cardboard layer, a weldable layer and a gas-tight layer,
- arranging, in at least the cardboard layer, at least one cut-out,
- forming a rounded container body from the blank by bending the blank such as to connect a first edge with an opposite edge of the blank wherein a joint is formed along the connected edges,
- applying a gas-tight and translucent material in such a way that the opening is covered by the gas-tight and translucent material.

The order of the method steps may be varied to some extent. The order presented above is useful for the method described further above where the cut-out is arranged also in the weldable and the gas-tight layer and where a strip of material, for the purpose of covering the joint or seam and the openings formed between the edges, is applied after having formed the container body. In this method: i) the at least one cut-out is arranged at a first edge of the blank; ii) an opening is formed at the at least one cut-out when forming the rounded container body, and iii) the gas-tight and translucent material is a strip of material that is applied along the joint along the connected edges.

However, the cut-out may alternatively be arranged only in the cardboard and the gas-tight (aluminium) layers before applying the weldable layer in the multilayer material. A weldable (and gas-tight and translucent) layer can then be applied onto the cardboard and the aluminium layers so as to cover the cut-out/opening that is placed at an edge of the blank. Further, the "edge" may in this case be an intended edge if the blank has not yet been cut out from a larger multilayer material. The particular part or strip of the weldable layer that covers the cut-out/opening can be made of a particular material with enhanced capability of being gas-tight. This means that only certain parts or strips of the weldable layer need to be made of this particular (and probably more expensive) material. Thus, in this case the step of "applying a gas-tight and translucent material..." is performed before the step of "forming a rounded container body from the blank by bending...". This variant of the method is also described further below.

In a further alternative method, the cut-out may be arranged only in the cardboard layer before applying any of the gas-tight or the weldable layers in the multilayer material. A gas-tight layer in the form of metalized film (that is sufficiently transparent to light) can then be applied onto the cardboard such as to cover the cut-out/opening. A weldable layer (which in this case does not have to be gas-tight) can then be applied onto the cardboard and the metalized layers so as to (together with the gas-tight layer) cover the cutout/opening that as in the alternative method above is placed at an edge of the blank. Further, the "edge" may in this case be an intended edge if the blank has not yet been cut out from a larger piece material. Thus, also in this case the step of "applying a gas-tight and translucent material..." is performed before the step of "forming a rounded container body from the blank by bending...".

The manufacturing method may also comprise one or several of the following steps:
- welding the material strip to the edges,
- fastening an end closure to the container body by generating an inductive welding energy for melting of the weldable layer; and
- transporting: a flow of body blanks to a container body forming unit; a flow of container bodies from the container body forming unit to a welding unit; and a flow of container bodies provided with end closures from the welding unit.

Figure 2 shows, in a schematic view, the principle of a first gas detection method applied to a container similar to what is shown in figure 1. Light emitted from a laser source is directed through the opening 2 into the headspace 7 of the filled and sealed container 1 (solid arrow). Scattered light exits the container 1 via the same window 2 (dashed arrow) and can be detected and analyzed. This method is suitable if the opening 2 is covered with a material that scatters the incoming light, i.e. if the covering material is translucent but not transparent to the incoming light.

Figure 3 shows, in a schematic view, the principle of a second gas detection method applied to a container similar to what is shown in figure 1, but in this example the container 1 also has a second window 2'. Light emitted from a laser source is directed through the opening 2 into the headspace 7 of the filled and sealed container 1 (solid arrow). Light propagates through the headspace 7 in the same direction and exits the container 1 via the second window 2' (dashed arrow) and can be detected and analyzed. This method is suitable if the opening 2 is covered with a material that is transparent to the incoming light. To improve the sensitivity of the method the light exiting the second window 2' can be reflected back towards the container 1 such as to pass through the head space once again and exit through the first opening 2 and then detected and analyzed.

How to detect and analyze the light, and how to determine the content of gas from such an analysis, is known as such to the person skilled in the art.

The method for detecting gas inside a sealed container of the type discussed here can be made "in-line", i.e. it can form part of the production line where the containers e.g. are filled and sealed (under an atmosphere of protection gas such as nitrogen), or it can be made "off-line", i.e. separate from the production line. If arranged "in-line" it can be arranged so that containers that e.g. contains too much oxygen are removed or marked in some way.

Thus, a production system comprising e.g. an apparatus for filling containers of the above type with a product and an apparatus for sealing the filled containers preferably also comprises an apparatus for carrying out the method for detecting gas inside the sealed container. Such a system preferably also comprises means for sorting out or marking containers that exhibit an abnormal gas content. The system may also comprise an apparatus for manufacturing the containers and an apparatus for applying an outer lid to the containers.

Figures 4a and 4b show examples of a blank 10 provided with a cut-out 9. In figure 4a the cut-out is placed at an edge of the blank 10. In this case the opening 2 is thus formed when forming the container 1, i.e. when bending the blank 10 to form a container similar to what is shown in figure 1.

In figure 4b the cut-out 9 is not positioned at the edge. This is possible when it is not intended to cover the cut-outs/openings with a (narrow) strip of material that also is intended to be used for joining the edges of the blank/container. Accordingly, positioning of the cut-out 9 as shown in figure 4b works fine if the cut-out/opening is to be covered by a gas-tight layer forming part of the multilayer structure. As described above this covering layer can be a metalized film (in which case the cut-out 9 is made only in the cardboard layer and where the metalized film functions as a gas-tight layer in the multilayered structure) or a weldable layer (in which case the cut-out 9 is made in both the cardboard layer and the gas-tight layer, which may be an aluminium layer, and where the weldable layer preferably contains zones or strips of particularly gas-tight material positioned such as to match the positions of the cut-outs/openings so that the weldable layer only partially needs to be made of such a particular material).

The above described methods for manufacturing the container 1 provide for a cost-effective production method where it is not needed to apply separate pieces of material only for the purpose of covering the openings.

The material covering the opening can form part of one of the laminated layers in a laminated multilayer structure. Typically, this would be the layer of weldable plastics in a cardboard-aluminium-plastics structure. To improve the gas barrier at the openings, the plastic material used can be prepared with strips of a material that forms a better gas barrier than normal weldable plastics, wherein the orientation and distance between the strips are adapted so that the strip covers the openings when the container is produced. For instance, this strip can be positioned in the laminating material so that it covers the (joint and) openings when a container is formed from a plane blank as described above. Preferably, the strip protrudes from one of the edges of the blank such as to overlap the other edge when the edges are connected. The gas barrier material strip can alternatively be formed of a separate piece of material applied onto the container such as to cover the at least one opening and, if appropriate, also the joint.

Generally, it is an advantage if the material(s) that cover the opening forms a barrier for UV-light (but still allows light for gas detection to pass) since the content of the container may be sensitive to UV-light.

## Claims

1. Method for manufacturing of a container (1) for food products or other sensitive products from blanks (10) of a cardboard based material, said method comprising the steps of:
- providing a substantially plane blank (10) of a multilayer material comprising a supporting cardboard layer, a weldable layer and a gas-tight layer,
- arranging, in at least the cardboard layer, at least one cut-out (2, 9),
- forming a rounded container body (4) from the blank (10) by bending the blank such as to connect a first edge with an opposite edge of the blank wherein a joint (8) is formed along the connected edges,
**characterized in**
**that** the at least one cut-out (2, 9) is arranged at the first edge of the blank (10), wherein an opening (2) is formed at the at least one cut-out when forming the rounded container body (4),
said method further comprising the step of:
- applying a gas-tight and translucent material in such a way that the opening (2) is covered by the gas-tight and translucent material.

2. Method according to claim 1, wherein the gas-tight and translucent material is a strip of material and said strip of material is applied along the joint (8) along the connected edges.

3. Method according to claim 1, wherein the gas-tight layer is an aluminium layer, wherein the cut-out (9) is arranged in the cardboard and the aluminium layers before applying the weldable layer in the multilayer material, wherein the weldable layer is a gas-tight and translucent layer and is applied onto the cardboard and the aluminium layers so as to cover the cut-out/opening (2, 9).

4. Method according to claim 1, wherein the gas-tight layer is a metalized film that is at least partly transparent to light, wherein the cut-out (9) is arranged in the cardboard layer before applying the gas-tight layer or the weldable layer in the multilayer material, wherein the gas-tight layer is applied onto the cardboard layer so as to cover the cut-out/opening (2, 9).

5. Method according to any one of claims 1-4, wherein the method further comprises one or several of the following steps:
- welding a material strip to the edges,
- fastening an end closure (6) to the container body (4) by generating an inductive welding energy for melting of the weldable layer,
- transporting: a flow of body blanks (10) to a container body forming unit; a flow of container bodies (4) from the container body forming unit to a welding unit; and a flow of container bodies (4) provided with end closures (6) from the welding unit.

## Patentansprüche

1. Verfahren zur Herstellung eines Behälters (1) für Nahrungsmittel oder andere empfindliche Produkte aus Rohlingen (10) aus einem kartonbasierten Material, wobei das Verfahren die folgenden Schritte umfasst:
- Bereitstellen eines im Wesentlichen ebenen Rohlings (10) aus einem mehrschichtigen Material, das eine unterstützende Kartonschicht, eine schweißbare Schicht und eine gasdichte Schicht umfasst,
- Anordnen, in zumindest der Kartonschicht, von zumindest einem Ausschnitt (2, 9),
- Formen eines runden Behälterkörpers (4) aus dem Rohling (10) durch Biegen des Rohlings, um eine erste Kante mit einer gegenüberliegenden Kante des Rohlings zu verbinden, wobei eine Verbindung (8) entlang der verbundenen Kanten geformt wird,
**dadurch gekennzeichnet, dass**
der zumindest eine Ausschnitt (2, 9) an der ersten Kante des Rohlings (10) angeordnet ist, wobei beim Formen des runden Behälterkörpers (4) eine Öffnung (2) an dem zumindest einen Ausschnitt geformt wird,
wobei das Verfahren weiter die folgenden Schritte umfasst:
- Aufbringen eines gasdichten und durchsichtigen Materials derart, dass die Öffnung (2) durch das gasdichte und durchsichtige Material bedeckt wird.

2. Verfahren nach Anspruch 1, wobei das gasdichte und durchsichtige Material ein Materialstreifen ist und der Materialstreifen entlang der Verbindung (8) entlang der verbundenen Kanten aufgebracht wird.

3. Verfahren nach Anspruch 1, wobei die gasdichte Schicht eine Aluminiumschicht ist, wobei der Ausschnitt (9) vor einem Aufbringen der schweißbaren Schicht in dem mehrschichtigen Material in den Karton- und den Aluminiumschichten angeordnet wird, wobei die schweißbare Schicht eine gasdichte und durchsichtige Schicht ist und auf die Karton- und die Aluminiumschichten aufgebracht wird, um den Ausschnitt/Öffnung (2, 9) zu bedecken.

4. Verfahren nach Anspruch 1, wobei die gasdichte Schicht ein metallisierter Film ist, der zumindest teilweise lichtdurchlässig ist, wobei der Ausschnitt (9) vor einem Aufbringen der gasdichten Schicht oder der schweißbaren Schicht in dem mehrschichtigen Material in der Kartonschicht angeordnet wird, wobei die gasdichte Schicht auf die Kartonschicht aufgebracht wird, um den Ausschnitt/Öffnung (2, 9) zu bedecken.

5. Verfahren nach einem der Ansprüche 1-4, wobei das Verfahren weiter einen oder mehrere der folgenden Schritte umfasst:
- Schweißen eines Materialstreifens an die Kanten,
- Befestigen eines Endverschlusses (6) an dem Behälterkörper (4) durch Erzeugen einer induktiven Schweißenergie zum Schmelzen der schweißbaren Schicht,
- Transportieren: eines Stroms von Körperrohlingen (10) zu einer Behälterkörperformungseinheit; eines Stroms von Behälterkörpern (4) von der Behälterkörperformungseinheit zu einer Schweißeinheit; und eines mit Endverschlüssen (6) versehenen Stroms von Behälterkörpern (4) von der Schweißeinheit.

## Revendications

1. Procédé de fabrication d'un récipient (1) pour produits alimentaires ou autres produits sensibles à partir d'ébauches (10) d'un matériau à base de carton, ledit procédé comprenant les étapes consistant à :
- fournir une ébauche sensiblement plane (10) d'un matériau multicouche comprenant une couche de carton de support, une couche soudable et une couche étanche au gaz,
- prévoir, dans au moins la couche de carton, au moins une découpe (2, 9),
- former un corps de récipient arrondi (4) à partir de l'ébauche (10) en pliant l'ébauche de façon à relier un premier bord avec un bord opposé de l'ébauche, dans lequel un joint (8) est formé le long des bords reliés l'un à l'autre,
**caractérisé en ce que**
ladite au moins une découpe (2, 9) est disposée sur le premier bord de l'ébauche (10), dans lequel une ouverture (2) est formée au niveau de ladite au moins une découpe, lors de la formation du corps de récipient arrondi (4),
ledit procédé comprenant en outre l'étape consistant à :
- appliquer un matériau étanche au gaz et translucide de sorte que l'ouverture (2) soit couverte par le matériau étanche au gaz et translucide.

2. Procédé selon la revendication 1, dans lequel le matériau étanche au gaz et translucide est une bande de matériau et ladite bande de matériau est appliquée le long du joint (8) le long des bords reliés l'un à l'autre.

3. Procédé selon la revendication 1, dans lequel la couche étanche au gaz est une couche en aluminium, dans lequel la découpe (9) est prévue dans le carton et les couches d'aluminium avant d'appliquer la couche soudable dans le matériau multicouches, dans lequel la couche soudable est une couche étanche au gaz et translucide, et est appliquée sur le carton et les couches en aluminium de façon à couvrir la découpe/l'ouverture (2, 9).

4. Procédé selon la revendication 1, dans lequel la couche étanche au gaz est un film métallisé qui est au moins partiellement transparent à la lumière, dans lequel la découpe (9) est prévue dans la couche en carton avant l'application de la couche étanche au gaz ou de la couche soudable dans le matériau multicouches, dans lequel la couche étanche au gaz est appliquée sur la couche en carton, de façon à couvrir la découpe/l'ouverture (2, 9).

5. Procédé selon l'une quelconque des revendications 1 à 4, dans lequel le procédé comprend en outre une ou plusieurs des étapes suivantes :
- soudage d'une bande de matériau aux bords,
- fixation d'une fermeture d'extrémité (6) au corps de récipient (4) en générant une énergie de soudage inductive pour faire fondre la couche soudable,
- transport : d'un flux d'ébauches de corps (10) à une unité de formation de corps de récipient ; d'un flux de corps de récipients (4) depuis l'unité de formation de corps de récipient à une unité de soudage ; et d'un flux de corps de récipients (4) dotés de fermetures d'extrémité (6) depuis l'unité de soudage.
